# EUROPEAN PATENT SPECIFICATION

(11) **EP 0 675 968 B1**
(45) Date of publication and mention of the grant of the patent: **13.03.2002**
(21) Application number: 93924928.0
(22) Date of filing: 14.10.1993
(51) Int. Cl.: C12Q 1/68, G01N 21/76, G01N 33/53, G01N 33/566

(54) **ENZYME-CATALYZED CHEMILUMINESCENT DETECTION OF PROTEINS AND NUCLEIC ACIDS USING HYDROXYARYL CYCLIC DIACYLHYDRAZIDE COMPOUNDS**
VON ENZYMEN KATALYSIERTE CHEMILUMINESZENTE DETEKTION VON PROTEINEN UND NUKLEINSÄUREN DURCH VERWENDUNG VON HYDROXYARYL-CYCLISCHEN DIACYLHYDRAZID-ZUSAMMENSETZUNGEN
DETECTION DE PROTEINES ET D'ACIDES NUCLEIQUES PAR CHIMIOLUMINESCENCE CATALYSEE PAR ENZYME, UTILISANT DES COMPOSES DIACYLHYDRAZIDE CYCLICLES D'HYDROXYARYLE

(30) Priority: 23.12.1992 US 996110
(43) Date of publication of application: 11.10.1995
(73) Proprietor: LUMIGEN, INC., Southfield, Michigan 48034 (US)
(72) Inventor: AKHAVAN-TAFTI, M., Hashem, Howell, Michigan 48834 (US); HANDLEY, Richard, S., Canton, MI 48187 (US)
(74) Representative: Jones, Helen Marjorie Meredith
(86) International application number: US9309820
(87) International publication number: WO9414979

(56) References cited:
- WO-A-94/10337
- GB-A- 2 237 383
- US-A- 4 794 073
- US-A- 4 927 769
- CHEMICAL ABSTRACTS, vol. 101, no. 20, 1984 Columbus, Ohio, US; abstract no. 182888e, KALINICHENKO I E ET AL: "Catalytic chemiluminescence reactions between substituted phthalic hydrizides and hexacyanoferrate" page 753; column left; XP002041457 & KALICHENKO ET AL.: ZH.ANAL KHIM, vol. 37, no. 7, - 1984 pages 1281-4,
- Journal of Immunological Methods, Volume 152, No. 2, issued 1992, E. HEINICKE et al., "Quantitative Dot-Blot Assay for Proteins Using Enhanced Chemiluminescence", pages 227-236, see entire document.

## Description

### BACKGROUND OF THE INVENTION

### (1) State of The Invention

This invention relates to a method of generating light chemically (chemiluminescence) in an assay on a surface such as a membrane by the action of a peroxidase enzyme with an oxidant such as hydrogen peroxide on a group of hydroxyaryl cyclic diacylhydrazides. The invention also relates to a method of greatly increasing the amount of chemiluminescence produced from this process by the use of specific enhancers and by conducting the reaction on a membrane. The invention also relates to the use of this method to detect the peroxidase enzyme. Further, the invention relates to the use of the method to detect and quantitate various biological molecules. For example, the method may be used to detect haptens, antigens and antibodies by the technique of immunoassay, proteins by Western blotting, DNA and RNA by Southern and Northern blotting, respectively. The method may also be used to detect RNA and DNA in dot blots, DNA in sequencing applications, and DNA in DNA probe assays.

### (2) Prior Art

a. Chemiluminescent Oxidation of Hydroxyaryl Cyclic Diacylhydrazides. A series of reports has appeared concerning the chemiluminescent reactions of 3-or 4-hydroxyphthalhydrazide with different chemical oxidizing agents (I. E. Kalinichenko, A. T. Pilipenko, V. A. Barovskii, Ukr. Khim. Zh. (Russ. Ed.), 43(10), 1102-6 (1977); Kalinichenko, I. E., Barovskii, V. A., Pilipenko, A. T., Ukr. Khim. Zh. (Russ. Ed.), 44(7), 748-52 (1978); A. T. Pilipenko, V. A. Barovskii, I. E. Kalinichenko, Zh. Anal. Khim., 33(10), 1880-4 (1978); I. E. Kalinichenko, V. A. Barovskii, Ukr. Khim. Zh. (Russ. Ed.), 45(1), 58-62 (1979); I. E. Kalinichenko, T. M. Tkachuk, A. T. Pilipenko, Zh. Anal. Khim., 39(7), 1281-4 (1984)). Gundermann reports the chemiluminescent oxidation of 7-hydroxynaphthalene-1,2-dicarboxylic hydrazide in aqueous solution with hydrogen peroxide and a metal catalyst (K.-D. Gundermann, W. Horstmann, G. Bergman, Lieb. Ann. der Chem, 684, 127-141 (1965)). No reports are known concerning the use of hydroxyaryl cyclic diacylhydrazides with a peroxidase enzyme or any enzyme to generate chemiluminescence. Further, there are no known reports of the use of hydroxyaryl cyclic diacylhydrazides to generate chemiluminescence for the detection of biological compounds.

b. Chemiluminescent Oxidation of Luminol and Related Compounds. Aminoaryl cyclic diacylhydrazides such as luminol and isoluminol react with H₂O₂ and a peroxidase enzyme catalyst (such as horseradish peroxidase, HRP) under basic conditions with emission of light. The reaction is also catalyzed by small amounts of several metal ions including Fe(III), Cu(II) and Cr(III) or iron-containing organic compounds (e.g. R. B. Brundett, D. F. Roswell, E. H. White, J. Am. Chem. Soc., 94, 7536 (1972)). This reaction has been used as the basis for analytical methods for the detection of H₂O₂ and for metal ions. Luminol and isoluminol may be directly conjugated to a species to be detected. The first chemiluminescent immunoassay using luminol as a label was reported by Schroeder for an assay of biotin. (H. R. Schroeder, P. O. Vogelhut, R. J. Carrico, R. C. Boguslaski, R. T. Buckler, Anal. Chem. 48, 1933 (1976)). Several applications of the use of luminol derivatives as labels have been reported since then (H. R. Schroeder in Luminescent Immunoassays: Perspectives in Endocrinology and Clinical Chemistry, M. Serio and M. Pazzagali, Eds., Raven Press, New York, pp 129-146 (1982); M. Pazzagli, G. Messeri, A. L. Caldini, G. Monetti, G. Martinazzo, M. Serio, J. Steroid Biochem., 19, 407 (1983); J. DeBoever, F. Kohen and D. Vandekerckhove in Bioluminescence and Chemiluminescence New Perspectivvees, J. Scholmerich, et al., Eds., J.Wiley & Sons, Chichester, pp 257-260 (1987). Various enhancers have also been employed in conjunction with horseradish peroxidase in the oxidation of luminol to increase the intensity of light emitted. These include, for example, D-luciferin (T. P. Whitehead, G. H. Thorpe, T. J. Carter, C. Groucutt, L. J. Kricka, Nature 305, 158 (1983)) and p-iodophenol and p-phenylphenol (G. H. Thorpe, L. J. Kricka, S. B. Mosely, T.P. Whitehead, Clin. Chem., 31, 1335 (1985)).

US 4,927,769 describes a method of enhancement of the chemiluminescence of an acridinium esters and to a enhanced chemiluminescent immunoassay, wherein said enhancers are selected from cationic surfactants, non ionic surfactants and sulphated primary alcohols.

Wurzberg *et al* teach a chemiluminescence method and a kit of luminol-like molecules. (Journal of Physical Chemistry, vol. 83, no. 21, pages 2687-2692)

US 4,614,712 discloses a chemiluminescent immunoassay with luciferase labelled ligands.

### C. Enzyme-Catalyzed Chemiluminescent Reactions

### (1) Enzymatic Generation of Hydrogen Peroxide.

Various enzymatic reaction schemes are known which produce hydrogen peroxide. The generated hydrogen peroxide can, in turn, be used to oxidize a compound which emits light. For example, glucose oxidase reacts with O₂ and sucrose to produce H₂O₂. Similarly, amino acid oxidase and cholesterol, respectively, and O₂ to produce H₂O₂. Examples of compounds which are oxidized by hydrogen peroxide to produce light are luminol and isoluminol, lucigenin, esters of N-methylacridine and esters or amides of oxalic acid. Glucose-6-phosphate dehydrogenase and galactose-6-phosphate dehydrogenase and galactose-6-phosphate dehydrogenase have been used to produce H₂O₂ indirectly by reduction of oxygen through an electron-relay system (k. Tanabe, T. Kawasaki, M. Maeda, A. Tsuji, M. Yabuuchi, Bunseki Kagaku, 36, 82 (1987); and A. Tsuji, M. Maeda, H. Arakawa, Anal. Sci., 5 497 (1989).

A method for detection of proteins in Western blots has been described, wherein the protein is immobilised on a nitrocelluose membrane and immunolabeled with horseradish peroxidase-conjugated secondary antibody. Horseradish peroxidase catalysis the oxidation of luminol, with the production of light (E. Heinicke *et al*, Journal of Immunological Methods vol. 152, no. 2, pages 227-236).

US 4,794,073 describes methods of detecting nucleic acid in samples, comprising: a) the immobilisation of nucleic acids in the sample; b) contacting the immobilised nucleic acids with a nucleotide probe having a base sequence substantially complementary to the sequence to be determined under conditions favorable to hydridisation between the probe and the sequence to be determined, said probe being labeled with a chemiluminescent label selected from the participants in an enhanced chemiluminescent reaction involving a 2,3-dihydro-1,4-phthalazinedione chemiluminescence precursor, a peroxidate enzyme and a chemiluminescent enhancer or comprising a binding site for a specific binding partner; c) separating resulting immobilised hybrids from probes which have not hybridised with the sequence to be determined, and where the probe comprising the binding site, adding the binding partner which is labeled with the chemiluminescent label; d) initiating the chemiluminescent reaction with the separated, labeled, immobilised hybrids, and e) detecting the resulting light emission.
Derivative. The compound o-aminophthalhydrazide-N-acetyl-β-D-glucosaminide (luminol-NAG) and 4'-(6'-diethylaminobenzofuranyl)phthalhydrazide-N-acetyl-β-D-glucosaminide are substrates for the enzyme N-acetyl-β-D-glucosaminidase which serve as a masked form of luminol. Upon action of the enzyme on these substrates, luminol or a luminol derivative are liberated which may be detected as described above (K. Sasamoto, Y. Ohkura, Chem. Pharm. Bull. 38(5), 1323 (1990); K. Sasamoto, Y. Ohkura, Chem. Pharm. Bull. 39(2), 411 (1991)).

d. Use of Chemiluminescence in DNA Hybridization assays. Biological assays such as enzyme immunoassays and DNA probe assays involving enzymes utilize a wide variety of substrates which either form a color (chromogenic), become fluorescent (fluorogenic) or emit light (chemiluminogenic) upon reaction with the enzyme. Of these three choices, chemiluminescence offers the greatest sensitivity. In an assay, the enzyme (reporter enzyme) is conjugated or bound to the molecule to be detected or to some other substance capable of selectively binding or associating with the molecule to be detected. Once the bound reporter enzyme is separated from unbound enzyme, a substrate is provided with which the reporter enzyme generates a signal. The enzyme horseradish peroxidase has been widely used in enzyme immunoassays and DNA hybridization assays with chemiluminescent detection using luminol or isoluminol as substrate (T. P. Whitehead, G. H. Thorpe, T. J. Carter, C. Groucutt, L. J. Kricka, Nature, 305, 158 (1983); G. H. Thorpe, L. J. Kricka, S. B. Mosely, T. P. Whitehead, Clin. Chem., 31, 1335 (1985); G. H. Thorpe, S. B. Mosely, L. J. Kricka, R. A. Stott, T. P. Whitehead, Anal. Chim. Acta, 170, 107 (1985); J. A. Matthews, A. Batki, C Hynds, L. J. Kricka, Anal. Biochem., 151, 205, (1985)). Commercially available kits for conjugation of HRP with enhanced luminol chemiluminescent detection are sold under the tradename "AMERLITE™" (Amersham International, PLC., Amersham, England).

### OBJECTS

It is therefore an object of the present invention to provide a method and hydroxyaryl cyclic diacylhydrazides for use in generating chemiluminescence by the action of a peroxidase enzyme and a phenol enhancer for the detection of biological materials and compounds. It is also an object of the present invention to provide a method and kit using hydroxyaryl cyclic diacylhydrazides on membranes for generating chemiluminescence by the action of a peroxidase enzyme and a phenol enhancer for the detection of peroxidase enzymes and enzyme-conjugates. Additionally, it is an object of the present invention to provide a method and kit using hydroxyaryl cyclic diacylhydrazides on surfaces such as membranes for generating chemiluminescence by the action of a peroxidase enzyme and a phenol enhancer for detection of nucleic acid assays in solution and on surfaces. Further, it is an object of the present invention to provide a method and kit using hydroxyaryl cyclic diacylhydrazides for generating chemiluminescence by the action of a peroxidase enzyme and a phenol enhancer, for detection of proteins in Western blots and nucleic acids in Northern blots, Southern blots and other DNA hybridization assays.

### IN THE DRAWINGS

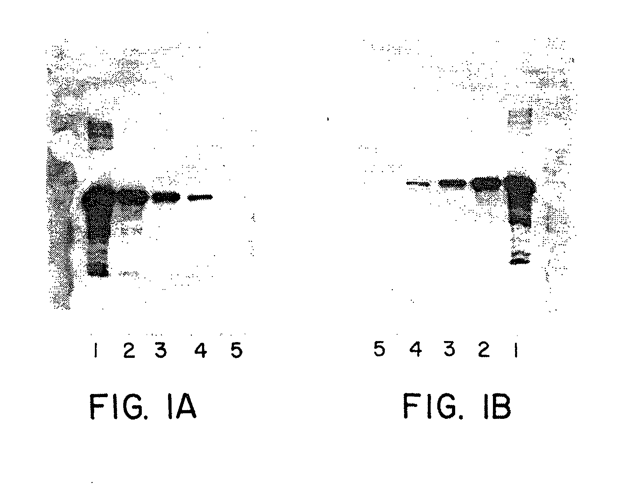

Figure 1 shows the result of a Western blot analysis of human transferrin on PVDF membrane (IMMOBILON P) with chemiluminescent detection using fractionated goat anti-human transferrin serum, rabbit anti-goat IgG-peroxidase conjugate, NaBO₃ and 5-hydroxy-2,3-dihydrophthalazine-1,4-dione. Human transferrin loaded into each slot was (1) 5000 pg, (2) 1000 pg, (3) 200 pg, (4) 50 pg and (5) 10 pg. The blots were exposed to X-OMAT AR x-ray film (A) for one minute after a 20 minute incubation or to OMC x-ray film (B) for 10 minutes after a 30 minute incubation.

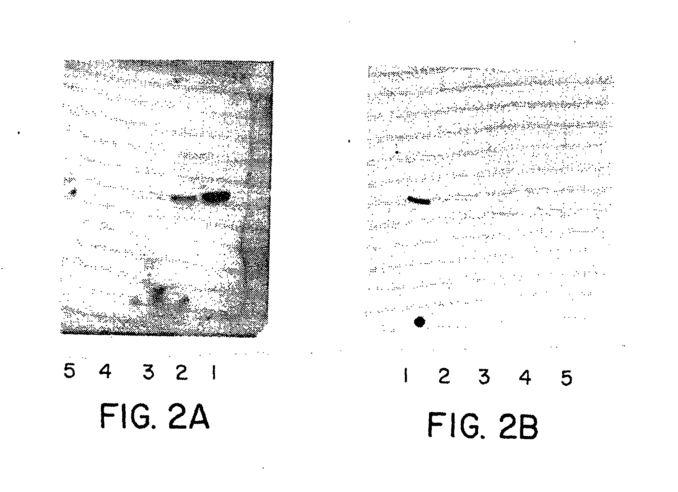

Figure 2 shows the result of a Western blot analysis of human transferrin on nitrocellulose with chemiluminescent detection using fractionated goat anti-human transferrin serum, rabbit anti-goat IgG-peroxidase conjugate, NaBO₃ and 5-hydroxy-2,3-dihydrophthalazine-1,4-dione. Human transferrin loaded into each slot was (1) 5000 pg, (2) 1000 pg, (3) 200 pg, (4) 50 pg and (5) 10 pg. The blots were exposed to X-OMAT AR x-ray film (A) for one minute after an 8 minute incubation or to OMC x-ray film (B) for 60 minutes after a 45 minute incubation.

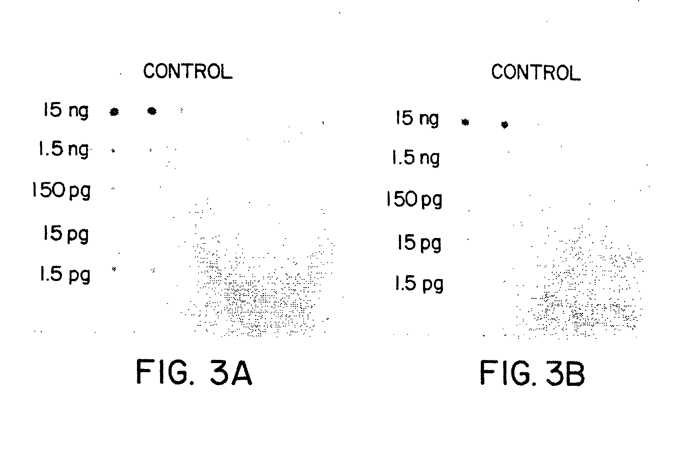

Figure 3 shows the. result of a dot blot analysis of biotinylated lambda DNA/Hind III fragments bound to avidin-horseradish peroxidase with chemiluminescent detection using a detection reagent containing 5-hydroxy-2,3-dihydrophthalazine-1,4-dione. Biotinylated lambda DNA/Hind III fragments in 6x SSC containing 100 µg/mL herring sperm DNA were dotted on nitrocellulose (A) or "IMMOBILON™-P" (B) in 15 ng, 1.5 ng, 150 pg, 15 pg and 1.5 pg quantities. 150 ng herring sperm DNA in 6x SSC was dotted. The blots were exposed to x-ray film for three minutes. The two columns of spots on the left in Figure 3 A and B represent dilutions of biotinylated lambda DNA/Hind III fragments containing 150 ng of herring sperm DNA. The control spots are only 150 ng of herring sperm DNA.

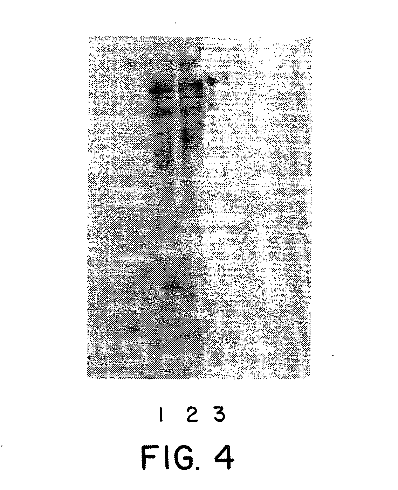

Figure 4 shows the result of a Southern blot analysis of a single copy gene, proto-oncogene mos with chemiluminescent detection of a horseradish peroxidase conjugated probe using a detection reagent containing 5-hydroxy-2,3-dihydrophthalazine-1,4-dione. The EcoR1 restricted mouse genomic DNA loaded in slots 1 and 2 was 20 micrograms. 95 ng of lambda DNA/Hind III fragments (Life Technologies, Inc.) was loaded in slot 3. The blots were exposed to x-ray film for two minutes.

### DESCRIPTION OF PREFERRED EMBODIMENTS

The present invention relates to an improvement in a method for detecting a member of a specific binding pair labeled with a peroxidase enzyme in an assay procedure by a chemiluminescent reaction, which comprises the chemiluminescent reaction caused by reacting with the binding pair on a membrane with a hydroxyaryl cyclic diacylhydrazide of the formula: 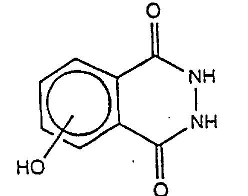 and a peroxide so that light is produced for detecting the analyte.

Further, the present invention relates to a kit for detecting a nucleic acid or a fragment of a nucleic acid comprising: an oligonucleotide probe complementary to the nucleic acid or a portion thereof labeled with a peroxidase enzyme; a hydroxyaryl cyclic diacylhydrazide compound of the formula: 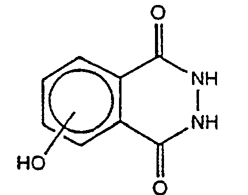 a phenolic enhancer compound in an amount which enhances light production from the diacylhydrazide in the presence of the peroxidase enzyme and decreases background chemiluminescence; a peroxide compound in an amount which reacts with the diacylhydrazide in the presence of the peroxidase; a chelating agent in an amount which prevents the peroxide compound from activating the diacylhydrazide prior to reaction with the peroxidase; and a water-soluble chemiluminescence-enhancing substance; and a membrane on which the hybridization is performed.

Further, the present invention relates to a kit for detecting a nucleic acid or a fragment of a nucleic acid comprising: an oligonucleotide probe complementary to the nucleic acid or a portion thereof labeled with an antigen; an antibody-peroxidase conjugate wherein the antibody is directed to the antigen; in admixture in an aqueous solution a hydroxyaryl cyclic diacylhydrazide compound of the formula: 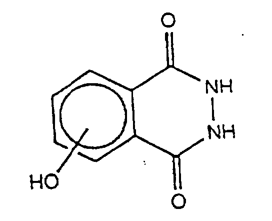 a phenolic enhancer compound in an amount which enhances light production from the diacylhydrazide in the presence of the peroxidase and decreases background chemiluminescence; a peroxide compound in an amount which reacts with the diacylhydrazide in the presence of the peroxidase; a chelating agent in an amount which prevents the peroxide compound from activating the diacylhydrazide prior to reaction with the peroxidase; and a water-soluble chemiluminescence-enhancing substance; and a surface, preferably a membrane, on which the hybridization is performed.

Finally, the present invention relates to a kit for detecting a protein comprising: an antibody-peroxidase conjugate wherein the antibody is directed to the protein or to a primary antibody directed to the protein; in admixture in an aqueous solution a hydroxyaryl cyclic diacylhydrazide compound of the formula: 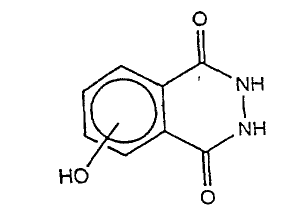 a phenolic enhancer compound in an amount which enhances light production from the diacylhydrazide in the presence of the peroxidase and decreases background chemiluminescence; a peroxide compound in an amount which reacts with the diacylhydrazide in the presence of the peroxidase; a chelating agent in an amount which prevents the peroxide compound from activating the diacylhydrazide prior to reaction with the peroxidase; a water-soluble chemiluminescence-enhancing substance; and a surface, preferably a membrane, on which the protein-antibody binding is performed.

The detection of chemiluminescence from the oxidation of a hydroxyaryl cyclic diacylhydrazide by hydrogen peroxide catalyzed by a peroxidase enzyme can be accomplished with good sensitivity. Enhancement of this reaction by incorporation of chemiluminescence-enhancing substances has permitted the measurement of chemiluminescence using still lower levels of the peroxidase enzyme. Coupling this enzyme to a biological molecule of interest then permits the detection of this biological molecule with great sensitivity.

A key consideration in developing ultrasensitive detection systems is to provide the largest signal possible through amplification while maintaining the lowest possible level of background signal in relation to the signal to be measured. For this purpose, additives have been discovered which suppress the generation of chemiluminescence from the reaction of hydrogen peroxide and hydroxyaryl cyclic diacylhydrazides in the absence of peroxidase enzymes.

The present invention involves a method of generating chemiluminescence from the oxidation of hydroxyaryl cyclic diacylhydrazides by the action of a peroxidase enzyme, a peroxide compound and enhancers. The invention also relates to the use of this method to detect the peroxidase enzyme with high sensitivity. Further, the invention relates to the use of the method to detect and quantitate various biological molecules which are bound to this enzyme by chemical bonds or through physical interactions. The intensity of the resulting chemiluminescence provides a direct measure of the quantity of labeled organic or biological molecule. For example, the method may be used to detect haptens, antigens and antibodies by the technique of immunoassay, proteins by Western blotting, DNA and RNA by Southern and Northern blotting, respectively. The method may also be used to detect DNA in DNA sequencing applications. The method may be used to detect hydrogen peroxide generated by enzymes such as glucose oxidase, glucose-6-phosphate dehydrogenase, galactose oxidase, galactose-6-phosphate dehydrogenase, and amino acid oxidase. The method may also therefore be used as a means to detect the enzymes mentioned above which generate hydrogen peroxide.

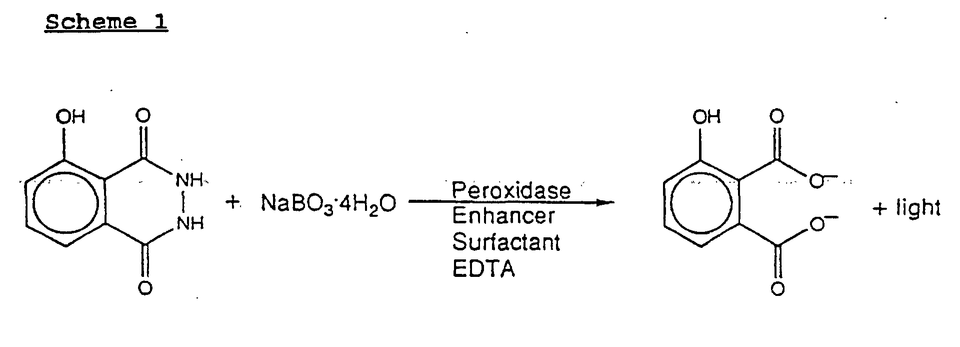

An important component of the invention is that a hydroxyaryl cyclic diacylhydrazide, a peroxide compound, a phenolic compound and a chelating agent such as EDTA may be combined in one solution and stored for later use without generating a large background chemiluminescent signal. This would not be expected in view of the fact that the combination of a hydroxyaryl cyclic diacylhydrazide and certain oxidants normally constitute a highly chemiluminescent reaction system.

An unexpected finding of the present invention is that incorporation of certain substituted phenol compounds in combination with surfactants into the reaction mixture decreases background chemiluminescence presence of added peroxidase. This is surprising in view of the fact that the substrate hydrazide is itself also a phenol. A second phenol would not a priori be expected to affect any enhancement. The combined suppressing of background chemiluminescence and enhancement of peroxidase-initiated chemiluminescence greatly extends the analytical range of use. Phenolic compounds found to enhance the amount of chemiluminescence produced in the reaction of hydroxyaryl cyclic diacylhydrazide with a peroxide compound and a peroxidase enzyme include, but are not limited to: p-phenylphenol, p-iodophenol, p-bromophenol, p-hydroxycinnamic acid, 6-bromo-2-naphthol, d-luciferin and 2-cyano-6-hydroxybenzothiazole. Selection of the best enhancer for a particular application will be based on performance, amount required, cost and availability.

The invention relates to a method for generating chemiluminescence by the action of a peroxidase enzyme or enzyme conjugate with a mixture containing a hydroxyaryl cyclic diacylhydrazide, a peroxide compound, an enhancer or catalyst and a suppressing agent in an aqueous buffer. The invention relates to a method for generating chemiluminescence by the action of a peroxidase enzyme or enzyme conjugate and a substrate for said peroxidase enzyme with a mixture containing a hydroxyaryl cyclic diacylhydrazide, a peroxide compound, a peroxidase enzyme, an enhancer or catalyst and a chelating agent in an aqueous buffer.

The invention also relates to the use of the method for the detection on solid supports or membranes such as nitrocellulose, nylon or polyvinylidene difluoride (PVDF) membranes, of proteins in Western blots, DNA in Southern blots and other DNA hybridization assays and RNA in Northern blots. For reasons which are not clear, the membrane significantly enhances the amount of light produced.

The present invention involves a solution in an aqueous buffer containing 1) a phenol enhancer, 2) a peroxide compound wherein the peroxide compound may be hydrogen peroxide, urea peroxide, or a perborate salt, 3) 5-hydroxy-2,3-dihydrophthalazine-1,4-dione, 4) a cation complexing agent wherein the agent may be selected from the group consisting of chelating agents such as ethylenediaminetetraacetic acid (EDTA), diethylenetriaminepentaacetic acid (DTPA), or ethylenebis(oxyethylenenitrilo)-tetraacetic acid (EGTA) and their salts, and 5) a surfactant chosen from either of two groups; the first group consisting of polymeric cationic surfactants selected from polymers bearing pendant phosphonium groups (such as described in EPA 92113448·2 or ammonium groups, the second group consisting of nonionic surfactants such as polyoxyethylenated alkylphenols, polyoxyethylenated alcohols, polyoxyethylenated ethers, polyoxyethylenated sorbitol esters and the like. It is anticipated that other hydroxyaryl cyclic diacylhydrazides may also be useful in practicing the present invention.

In a preferred method of practicing the present invention an aqueous buffer solution with a pH in the range of 8-10 containing 5-hydroxy-2,3-dihydrophthalazine-1,4-dione at a final concentration from about 0.01 M to 1 x 10⁻⁴ M, a phenol compound such as p-iodophenol at a final concentration from about 0.01 M to 1 x 10⁻⁶ M and a surfactant at a final concentration from about 1% to 0.01% (v/v) is mixed with a second solution in water or aqueous buffer containing a peroxide source such as hydrogen peroxide or, preferably, a perborate salt and a cation complexing agent such as EDTA at a final concentration from about 1 x 10⁻³ M to 1 x 10⁻⁶ M to form the detection reagent solution. This solution is contacted with the peroxidase enzyme which may either be in solution or adhered to a solid support. Optimum concentrations of reagents must be determined individually for each composition. The concentration of enhancer in particular should be optimized with care for each different enhancer used in order to produce the maximum enhancement of light emission. The choice of the polymeric surfactant is dictated by the application, especially by the membrane on which the chemiluminescent reaction is to be performed. A cationic surfactant and especially a phosphonium salt containing polymer is preferred when performing the reaction on nitrocellulose.

Significant advantages of hydroxyaryl cyclic diacylhydrazides and compositions of the present invention containing them is their stability toward thermal, hydrolytic and photochemical degradation and ease of purification. Aminoaryl cyclic diacylhydrazides such as luminol and compositions containing them are readily decomposed by room light leading to loss of sensitivity and poor reproducibility when used in chemiluminescence detection schemes (Y. Omote, H. Yamamoto, N. Sugiyama, Chem. Commun., 914 (1970)). Aminoaryl cyclic diacylhydrazides are difficult to prepare and maintain in a state of high purity and must either be protected from light or purified immediately before use (R. A. W. Stott, L. J. Kricka, in Bioluminescence and Chemiluminescence New Perspectives, J. Scholmerich et al, Eds., John Wiley & Sons, Chichester, England, 237-240 (1987)). Hydroxyaryl cyclic diacylhydrazides can be easily purified to a high degree by recrystallization and maintain their purity without special storage requirements. Aqueous solutions of 5-hydroxy-2,3-dihydrophthalazine-1,4-dione retain their activity after storage at ambient temperature and in room light for at least two months.

### 1. Chemiluminescent Detection of Proteins by Western Blot

Rabbit anti-goat IgG-peroxidase conjugate and rabbit anti-goat IgG-peroxidase were obtained from Cappel Products (Durham, N.C.). Human transferrin and fractionated goat anti-human transferrin serum were purchased from Sigma Chemical Co. (St. Louis, MO). The IgG sample was centrifuged at 10,000 g for two minutes and the supernatant was used in the immunological reaction. "IMMOBILON™-P" transfer membrane was obtained from Millipore Corp. (Bedford, MA). Kodak (Rochester, NY) X-OMAT AR and OMC films were used in the assay procedure.

SDS-PAGE was performed utilizing the buffer system described by Laemmli (U. K. Laemmli, Nature (London), 227,680 (1970)). The stacking gel was 4.38% acrylamide:0.12% bisacrylamide. The separating gel was 6.81% acrylamide:0.19% bisacrylamide. Following electrophoresis the gel was equilibrated for 7-8 minutes with the transfer buffer which contained 20 mM Tris, 153 mM glycine and 20% (v/v) methanol. The gel, sandwiched between a sheet of transfer membrane and a sheet of chromatography paper 3MM (Whatman), was placed in the transfer unit (Bio-Rad Laboratories, Richmond, CA). The proteins in the gel were electroeluted for 50-60 minutes at 4°C at a 100 V constant voltage. The membrane was then placed in 50 mM Tris-HCl buffered saline at pH 7.4 (TBS) at 4°C overnight. After this period the membrane was washed with TBS for 15 minutes.

The membrane was treated with 0.05% Tween-20 in 50 mM Tris-HCl buffered saline at pH 7.4 (T-TBS) containing 1% non-fat powdered milk (NFM) for one hour at room temperature. This blocked membrane was incubated for 75 minutes at room temperature with primary antibody (1:500 dilution of goat anti-human transferrin IgG fraction) using T-TBS containing 1% NFM.

The membrane was then rinsed and washed three times for ten minutes each with T-TBS at room temperature. The washed membrane was incubated for one hour at room temperature with secondary antibody (1:25000 dilution of rabbit anti-goat IgG peroxidase conjugate) using T-TBS containing 1% NFM. The membrane was rinsed and washed four times for ten minutes each with T-TBS followed by a ten minute wash with TBS.

The washed membrane was soaked in a detection reagent solution containing a peroxide compound and 5-hydroxy-2,3-dihydrophthalazine-1,4-dione for ten minutes, drained, placed between sheets of transparency film. The X-ray film was exposed to the membrane for one to ten minutes and developed.

Composition of detection reagent solution:

| Solution A | Tris buffer, pH 8.8 | 0.1M |
|---|---|---|
| | 5-hydroxy-2,3-dihydrophthalazine -1,4-dione | 3.0 x 10⁻³M |
| | p-iodophenol | 4.8 x 10⁻³M |
| | Tween 20 | 1.0% (w/w) |

| Solution B | Tris buffer, pH 8.8 | 0.1M |
|---|---|---|
| | NaBO₃.4H₂O | 6.5x10⁻³M |
| | EDTA | 1x10⁻⁴M |

The reagent is prepared by mixing solutions A and B in a 1:1 ratio.

To determine the sensitivity of this detection system for Western blotting, a model system of transferrin was used to provide polypeptide bands in known quantities. The transferrin standards utilized were clearly visible down to 10 pg/slot without background after a one minute exposure to Kodak X-OMAT AR x-ray film (Figure 1A) or after a ten minute exposure to OMC x-ray film (Figure 1B). It was possible to make several exposures of the membrane during the first hour as the membrane continued to emit light.

Chemiluminescent detection of proteins on Western blots using the system of anti-goat IgG-peroxidase conjugate, peroxide and 5-hydroxy-2,3-dihydrophthalazine-1,4-dione is capable of detecting extremely small amounts of protein with very short exposure times. Under optimum conditions ten picograms of protein can be detected on x-ray film with an exposure time of one minute using X-OMAT AR or ten minutes using OMC.

### 2. Chemiluminescent Western Blot on Nitrocellulose Membrane.

A Western blot analysis was performed according to the protocol described in Example 1 with the substitution of nitrocellulose membrane for PVDF and with a detection reagent solution prepared by mixing solutions A and B below in a 1:1 ratio. Bovine serum albumin was substituted for non-fat milk in the blocking solution.

| Solution A | |
|---|---|
| Tris buffer, pH 8.8 | 0.1M |
| 5-hydroxy-2,3-dihydrophthalazine-1, 4-dione | 3.0x10⁻³M |
| p-iodophenol | 4.8x10⁻³ M |
| poly(vinylbenzyl)tributylphosphonium chloride-poly(vinylbenzyl) trioctylphosphonium chloride copolymer | 0.05% (w/w) |
| (Tween 20) polyoxyethylene sorbitan monolaurate | 1.0% w/w |

| Solution B | |
|---|---|
| Tris buffer, pH 8.8 | 0.1M |
| NaBO₃.H₂O | 6.5x10⁻³M |
| EDTA | 1x10⁻⁴M |

### 3. Chemiluminescent Detection of DNA Dot Blots

"IMMOBILON™-P" (Millipore, Bedford, MA) was sequentially soaked in methanol for 40 seconds, in H₂O for two minutes and in 6x SSC (20x SSC is 3 M NaCl, 0.3 M sodium citrate, pH 7.0) for five minutes. The membrane was placed on two thicknesses of 3MM blotting paper (Whatman) containing 6x SSC. Biotinylated lambda DNA/Hind III fragments (Life Technologies, Inc., Bethesda, MD) were diluted with 6x SSC containing 100 µg/mL sheared herring sperm DNA (Boehringer-Mannheim, Indianapolis, IN) and 1.5 µL of each solution of DNA was dotted on the "IMMOBILON™-P" membrane or on nitrocellulose membrane (Schleicher & Schuell). Blots were air dried for 30 minutes and then baked for one hour at 80°C. Baked "IMMOBILON™-P" was sequentially soaked in methanol for ten seconds, in H₂O for two minutes and in 50 mM Tris-HCl buffered saline at pH 7.4 (TBS) for ten minutes. Baked nitrocellulose membrane was sequentially soaked in H₂O for two minutes and in TBS for five minutes.

The membranes were blocked with 0.05% Tween-20 in Tris-HCl buffered saline at pH 7.4 (T-TBS) containing 1% non-fat milk (NFM) for one hour at room temperature with gentle agitation and then raised twice with T-TBS. Blots were incubated with a 1:10,000 dilution of avidin-horseradish peroxidase (Cappel Research Products) in T-TBS for 15 minutes at room temperature with gentle agitation. The membrane was then rinsed four times for 15 minutes each with T-TBS followed by four rinses with H₂O. Excess solution was removed from the membrane, the membrane was transferred to a fresh container and then incubated with the reagent solution of Example 1 for five minutes. Excess detection reagent was drained off and the membrane was placed between transparency film (Arkwight, Inc.) followed by autoradiography using Kodak X-OMAT XAR 5 film.

The DNA dot blots in Figure 3 were performed with avidin-horseradish peroxidase and reagents of the present invention using (1) 15 ng, (2) 1.5 ng, (3) 150 pg, (4) 15 pg, (5) 1.5 pg and (6) 0 pg quantities of biotinylated lambda DNA/Hind III fragments each containing 150 ng herring sperm DNA. Spots of 15 ng biotinylated lambda DNA/Hind III fragments on nitrocellulose membrane produced stronger signals than the control spot containing 150 ng herring sperm DNA (Figure 3A). The observed detection limit of at least 15 ng biotinylated lambda DNA dotted on nitrocellulose membrane was also obtained with dot blots on Immobilon™-P using this detection reagent (Figure 3B). Longer x-ray film exposures were required when using Immobilon™-P with either detection reagent (data not shown) to achieve equivalent signal. These observations demonstrate that chemiluminescent detection of DNA dot blots utilizing reagents of the present invention yield excellent sensitivity with short exposure times.

### 4. Chemiluminescent Detection of Southern Blots

Mouse genomic DNA (Clontech Laboratories, Inc., Palo Alto, California) was cleaved to completion with restriction endonuclease EcoR1 (Boehringer-Mannheim, Indianapolis, Indiana) at a concentration of 50 µg/mL. The restricted DNA was purified by extraction once with phenol/chloroform, once with chloroform and was precipitated with ethanol. The purified DNA was separated by 0.8% agarose gel electrophoresis. The electrophoresis buffer was 40 mM Tris-acetate and 2 mM EDTA (pH 8.0). After electrophoresis the gel was rinsed with H₂O and then soaked in 0.25 N HCl for 12 minutes with gentle agitation. The gel was once again rinsed with H₂O and incubated twice in 0.4 M NaOH/0.6 M NaCl for 15 and 20 minutes, respectively.

"IMMOBILONT"-P" was soaked sequentially in methanol, H₂O and 0.4 M NaOH/0.6 M NaCl for 40 seconds, 2 and 10 minutes, respectively. The DNA in the alkaline treated agarose gel was transferred onto alkaline treated "IMMOBILON™-P" by capillary blotting overnight using 0.4 M NaOH/0.6 M NaCl. The membrane was neutralized by agitation in 0.75 M NaCl/0.5 M Tris-HCl (pH 7.5) for 10 minutes at room temperature followed by agitation in 6x SSC (20x SSC is 3 M NaCl, 0.3 M sodium citrate, pH 7.0) three times for five minutes each at room temperature. The blots were air-dried on 3MM blotting paper for two hours. The membrane was UV-irradiated for two minutes with a Fotodyne Model 3-3000 illuminator at a distance of 20 cm.

The membrane was soaked in methanol for five seconds and then in H₂O for two minutes to remove air in the membrane, followed by prehybridization using hybridization buffer ("AMERSHAM #RPN.3000", Amersham International, PLC, Amersham, England) containing 0.5 M NaCl and blocking agents (Amersham #RPN.3000) for 60 minutes at 42°C. The hybridization probe, v-mos DNA (Clontech Lab. Inc., Palo Alto, California) was labeled with horseradish peroxidase (Cappel, Durham, North Carolina) according to the manufacturer's instructions and the hybridization proceeded overnight at 42°C using a hybridization buffer containing 0.5 M NaCl, blocking agents and 300 ng/mL horseradish peroxidase labeled v-mos DNA. The membrane was washed sequentially with 0.5x SSC/0.4% SDS twice for five minutes each at room temperature, with 0.5x SSC/0.4% SDS three times for ten minutes each at 55°C, with 2x SSC twice for five minutes each at room temperature and then with H₂O another four times. The membrane was placed on 3MM blotting paper for one minute to remove excess solution, then transferred to a clean container followed by the addition of the detection reagent of Example 1. After a one minute incubation, excess solution was drained off and the blots were placed between sheets of transparency film followed by exposure to Kodak X-OMAT XAR 5 film for 2 minutes.

The reagent of the present invention can be used to detect a single copy gene in mouse genomic DNA as shown in Figure 4. The v-mos probe, which is complementary to the proto-oncogene mos, was hybridized to Southern blotted mouse genomic DNA. The target restriction fragment is 1.4 kd providing 8.4 pg (1.1 x 10⁻¹⁷ moles) of target DNA in the 20 µg leading tracks. Close examination of the x-ray film indicates that two additional bands can be found in lanes 1 and 2. The artifactual bands could possibly be associated with alkaline transfer. Lambda DNA/Hind III fragments were visible using the reagent of the present invention. The amount of each Hind III fragment is at least 470 times higher than that of the single copy gene. High levels of DNA fragments should yield non-specific signals in Southern blots using this detection reagent. This conclusion is supported by the results from the DNA dot blots described above. It is apparent that a single copy gene can be clearly detected using this detection reagent after a short exposure to x-ray film.

It will be appreciated that the foregoing examples are illustrative only and are not taken to be limiting unless so stated. Those skilled in the art will recognize that modifications and variations of the examples described above can be made without departing from the spirit of the invention. The present invention is limited only by the claims appended below.

The term surfactant indicates a water-soluble surface active agent selected from cationic, non-ionic, zwitterionic, and anionic compounds. Included are poloxyethylene glycol ethers, ammonium and phosphonium salts and the like.

## Claims

1. A method for detecting a member of a specific binding pair labeled with a peroxidase enzyme in an assay procedure by a chemiluminescent reaction, comprising:
(a) reacting with the binding pair on a surface with a hydroxyaryl cyclic diacylhydrazide of the formula: 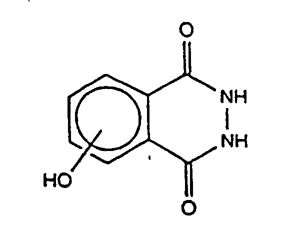
and a peroxide so that light is produced for detecting the analyte.

2. The method of Claim 1 wherein the specific binding pair consists of a nucleic acid and an oligonucleotide probe which is complementary to and binds to the target nucleic acid or some portion thereof, wherein the probe is associated with the peroxidase enzyme either through a covalent bond, physical attractions, or by linkage through an enzyme-labeled specific binding pair.

3. The method of Claim 2 wherein the nucleic acid is DNA.

4. The method of Claim 2 wherein the nucleic acid is RNA.

5. The method of Claim 2 wherein the peroxidase enzyme is horseradish peroxidase.

6. The method of Claim 2 additionally consisting of the step of (a) hybridizing the nucleic acid and labeled oligonucleotide probe directly labeled with the horseradish peroxidase to form the binding pair, and (b) reacting the binding pair with the hydroxyaryl cyclic diacylhydrazide and a peroxide so that light is produced.

7. The method of Claim 2 additionally consisting of (a) hybridizing the nucleic acid and labeled oligonucleotide probe to form the binding pair, (b) reacting the binding pair with a substance which strongly binds to the pair and which is bound to the peroxidase enzyme to form a bound enzyme conjugate, and (c) reacting the bound enzyme conjugate with a hydroxyaryl cyclic diacylhydrazide and a peroxide so that light is produced.

8. The method of Claim 7 wherein the binding pair contains biotin and the substance is selected from the group consisting of an avidin-horseradish peroxidase conjugate and a streptavidin-peroxidase conjugate.

9. The method of Claim 7 wherein the pair contains an antigenic site and the substance is an antibody-peroxidase conjugate which binds to the site.

10. The method of claim 1 wherein the specific binding pair consists of an antigen and an antibody which binds to the antigen.

11. The method of Claim 10 wherein the antigen or antibody is associated with a peroxidase enzyme either through a covalent bond, physical attractions, or by linkage through an enzyme-labeled specific binding pair.

12. The method of claim 11 wherein the enzyme is horseradish peroxidase.

13. The method of Claim 12 additionally consisting of (a) forming an antigen-antibody complex and (b) reacting the antigen-antibody complex which is labeled with a peroxidase enzyme with the hydroxyaryl cyclic diacylhydrazide and a peroxide so that light is produced.

14. The method of Claim 11 additionally consisting of (a) forming an antigen-antibody complex; and (b) reacting the labeled antigen-antibody complex with a substance which strongly binds to the pair and is bound to a peroxidase enzyme, and (c) reacting the bound enzyme conjugate with the hydroxyaryl cyclic diacylhydrazide and a peroxide so that light is produced.

15. The method of Claim 14 wherein the pair is labeled with biotin and the substance is selected from the group consisting of an avidin-peroxidase conjugate and streptavidin-peroxidase conjugate.

16. The method of Claim 14 wherein the pair is labeled with a second antigen which reacts with the antibody and the label-binding substance is a conjugate between a peroxidase enzyme and an antibody to the second antigen.

17. The method of any one of Claims 1, 6, 7, 13 or 14 wherein the light is produced in a reaction using the peroxidase enzyme and the hydroxyaryl cyclic diacylhydrazide in a composition which comprises:
the hydroxyaryl cyclic diacylhydrazide; a phenolic compound which enhances light production from the diacylhydrazide and decreases light production from the diacylhydrazide in the absence of the peroxidase; a peroxide compound which reacts with the diacylhydrazide in the presence of the peroxidase; a chelating agent which prevents the peroxide compound from activating the diacylhydrazide prior to the addition of the peroxidase to the composition; and a water-soluble surfactant compound which acts to enhance the light production.

18. The method of Claim 17 wherein the water-soluble surfactant compound is a non-ionic surfactant.

19. The method of Claim 17 wherein the water-soluble surfactant is a cationic polymer.

20. The method of Claim 19 wherein the cationic polymer is a polymeric phosphonium salt selected from poly(vinylbenzyl)trialkylphosphonium salts.

21. The method of Claim 19 wherein the cationic polymer is a polymeric ammonium salt selected from poly(vinylbenzyl)trialkylammonium salts.

22. The method of any one of Claims 1, 6, 7, 13, 14 or 17 wherein the hydroxyaryl cyclic diacylhydrazide is 5-hydroxyphthalazine-1,4-dione.

23. The method of Claim 1 wherein the surface is selected from the group consisting of nylon, nitrocellulose and polyvinylidene difluoride membranes.

24. The method of any one of Claims 1, 6, 7, 13, 14 or 17 wherein the light produced is detected on a photographic film.

25. A kit for detecting a nucleic acid or a fragment of a nucleic acid comprising:
(a) an oligonucleotide probe complementary to the nucleic acid or a portion thereof labeled with a peroxidase enzyme;
(b) a hydroxyaryl cyclic diacylhydrazide compound of the formula: 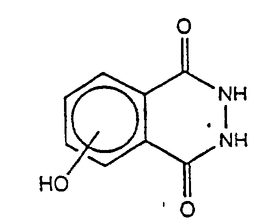 a phenolic enhancer compound in an amount which enhances light production from the diacylhydrazide in the presence of the peroxidase enzyme and decreases background chemiluminescence;
(c) a peroxide compound in an amount which reacts with the diacylhydrazide in the presence of the peroxidase;
(d) a chelating agent in an amount which prevents the peroxide compound from activating the diacylhydrazide prior to reaction with the peroxidase; and a water-soluble chemiluminescence-enhancing surfactant; and
(e) a membrane on which the hybridization is performed.

26. A kit for detecting a nucleic acid or a fragment of a nucleic acid comprising:
(a) an oligonucleotide probe complementary to the nucleic acid or a portion thereof labeled with biotin;
(b) a streptavidin-peroxidase or avidin-peroxidase conjugate;
(c) in admixture in an aqueous solution a hydroxyaryl cyclic diacylhydrazide compound of the formula: 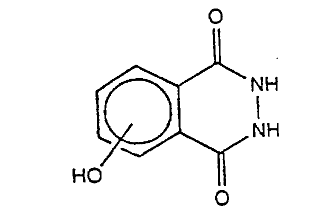 a phenolic enhancer compound in an amount which enhances light production from the diacylhydrazide in the presence of the peroxidase and decreases background chemiluminescence; a peroxide compound in an amount which reacts with the diacylhydrazide in the presence of the peroxidase; a chelating agent in an amount which prevents the peroxide compound from activating the diacylhydrazide prior to reaction with the peroxidase; and a water-soluble chemiluminescence-enhancing surfactant; and
(d) a membrane on which the hybridization is performed.

27. A kit for detecting a nucleic acid or a fragment of a nucleic acid comprising:
(a) an oligonucleotide probe complementary to the nucleic acid or a portion thereof labeled with an antigen; an antibody-peroxidase conjugate wherein the antibody is directed to the antigen;
(b) in admixture in an aqueous solution a hydroxyaryl cyclic diacylhydrazide compound of the formula: 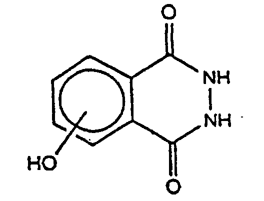 a phenolic enhancer compound in an amount which enhances light production from the diacylhydrazide in the presence of the peroxidase and decreases background chemiluminescence; a peroxide compound in an amount which reacts with the diacylhydrazide in the presence of the peroxidase; a chelating agent in an amount which prevents the peroxide compound from activating the diacylhydrazide prior to reaction with the peroxidase; and a water-soluble chemiluminescence-enhancing surfactant; and
(c) a membrane on which the hybridization is performed.

28. A kit for detecting a protein comprising:
(a) an antibody-peroxidase conjugate wherein the antibody is directed to the protein or to a primary antibody directed to the protein;
(b) in admixture in an aqueous solution a hydroxyaryl cyclic diacylhydrazide compound of the formula: 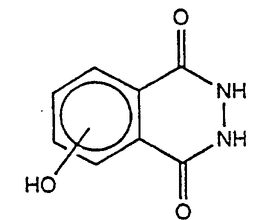 a phenolic enhancer compound in an amount which enhances light production from the diacylhydrazide in the presence of the peroxidase and decreases background chemiluminescence; a peroxide compound in an amount which reacts with the diacylhydrazide in the presence of the peroxidase; a chelating agent in an amount which prevents the peroxide compound from activating the diacylhydrazide prior to reaction with the peroxidase; a water-soluble chemiluminescence-enhancing surfactant; and
(c) a membrane on which the protein-antibody binding is performed.

29. A kit for detecting a protein comprising:
(a) a primary antibody directed to the protein; a peroxidase-secondary antibody conjugate directed to the primary antibody;
(b) in admixture in an aqueous solution a hydroxyaryl cyclic diacylhydrazide compound of the formula: 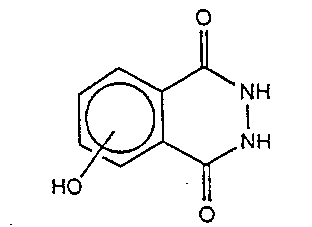 a phenolic enhancer compound in an amount which enhances light production from the diacylhydrazide in the presence of the peroxidase and decreases background chemiluminescence; a peroxide compound in an amount which reacts with the diacylhydrazide in the presence of the peroxidase; a chelating agent in an amount which prevents the peroxide compound from activating the diacylhydrazide prior to reaction with the peroxidase; a water-soluble chemiluminescence-enhancing surfactant and a membrane on which the protein-antibody binding is performed.

30. The kit of any of Claims 25 to 29 wherein the hydroxyaryl cyclic diacylhydrazide compound is 5- hydroxy-2,3-dihydrophthalazine-1,4-dione.

31. The kit of any of Claims 25 to 29 wherein the water-soluble chemiluminescence-enhancing surfactant is a non-ionic surfactant.

32. The kit of any of Claims 25 to 29 wherein the water-soluble chemiluminescence-enhancing surfactant is a cationic polymer.

33. The kit of Claim 32 wherein the cationic polymer is a polymeric phosphonium salt.

34. The kit of Claim 32 wherein the cationic polymer is a polyvinylbenzyl trialkylphosphonium salt.

35. The kit of Claim 32 wherein the cationic polymer is a polymeric ammonium salt.

36. The kit of Claim 32 wherein the cationic polymer is a polyvinylbenzyl trialkylammonium salt.

## Patentansprüche

1. Verfahren zum Nachweis eines Elementes eines spezifischen, mit einem Peroxidaseenzym markierten Bindungspaares in einem Analyseverfahren mittels Chemolumineszenzreaktion, das Folgendes umfasst:
(a) Umsetzung mit dem Bindungspaar auf einer Oberfläche mit einem zyklischen Hydroxyaryl-Diacylhydrazid der Formel: 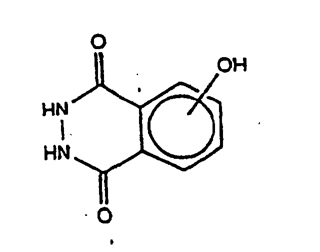
und einem Peroxid zur Erzeugung von Licht zum Nachweis des Analyts.

2. Verfahren nach Anspruch 1, worin das spezifische Bindungspaar aus einer Nukleinsäure und einer Oligonukleotidsonde besteht, die zu der Ziel-Nukleinsäure oder einem Teil davon komplementär ist und sich an sie bindet, wobei die Sonde mit dem Peroxidaseenzym entweder durch eine kovalente Bindung, physikalische Anziehung oder eine Verknüpfung über ein enzymmarkiertes spezifisches Bindungspaar assoziiert ist.

3. Verfahren nach Anspruch 2, worin die Nukleinsäure DNA ist

4. Verfahren nach Anspruch 2, worin die Nukleinsäure RNA ist.

5. Verfahren nach Anspruch 2, worin das Peroxidaseenzym Meerrettichperoxidase ist.

6. Verfahren nach Anspruch 2, das zusätzlich aus dem Schritt der (a) Hybridisierung der Nukleinsäure und der direkt mit der Meerrettichperoxidase markierten Oligonukleotidsonde zur Bildung des Bindungspaares, und (b) der Umsetzung des Bindungspaares mit dem zyklischen Hydroxyaryl-Diacylhydrazid und einem Peroxid zur Erzeugung von Licht besteht.

7. Verfahren nach Anspruch 2, das zusätzlich aus (a) der Hybridisierung der Nukleinsäure und der markierten Oligonukleotidsonde zur Bildung eines Bindungspaares, (b) der Umsetzung des Bindungspaares mit einer Substanz, die sich stark an das Paar bindet und an das Peroxidaseenzym gebunden ist, zur Bildung eines verbundenen Enzymkonjugates und (c) der Umsetzung des verbundenen Enzymkonjugates mit einem zyklischen Hydroxyaryl-Diacylhydrazid und einem Peroxid zur Erzeugung von Licht besteht.

8. Verfahren nach Anspruch 7, worin das Bindungspaar Biotin enthält und die Substanz ausgewählt ist aus der Gruppe bestehend aus einem Avidin-Meerrettichperoxidase-Konjugat und einem Streptavidin-Peroxidase-Konjugat.

9. Verfahren nach Anspruch 7, worin das Paar eine Antigenstelle enthält und die Substanz ein Antikörper-Peroxidase-Konjugat ist, das sich an die Stelle bindet.

10. Verfahren nach Anspruch 1, worin das spezifische Bindungspaar aus einem Antigen und einem sich an das Antigen bindenden Antikörper besteht.

11. Verfahren nach Anspruch 10, worin das Antigen oder der Antikörper mit dem Peroxidaseenzym entweder durch eine kovalente Bindung, physikalische Anziehung oder eine Verknüpfung über ein enzymmarkiertes spezifischen Bindungspaar assoziiert ist.

12. Verfahren nach Anspruch 11, worin das Enzym Meerrettichperoxidase ist.

13. Verfahren nach Anspruch 12, das zusätzlich aus (a) der Bildung eines Antigen-Antikörper-Komplexes und (b) der Umsetzung des mit einem Peroxidaseenzym markierten Antigen-Antikörper-Komplexes mit dem zyklischen Hydroxyaryl-Diacylhydrazid und einem Peroxid zur Erzeugung von Licht besteht.

14. Verfahren nach Anspruch 11, das zusätzlich aus (a) der Bildung eines Antigen-Antikörper-Komplexes, (b) der Umsetzung des markierten Antigen-Antikörper-Komplexes mit einer Substanz, die sich stark an das Paar bindet und an ein Peroxidaseenzym gebunden ist, und (c) der Umsetzung des verbundenen Enzymkonjugats mit dem zyklischen Hydroxyaryl-Diacylhydrazid und einem Peroxid zur Erzeugung von Licht besteht.

15. Verfahren nach Anspruch 14, worin das Paar mit Biotin markiert ist und die Substanz ausgewählt ist aus der Gruppe bestehend aus einem Avidin-Peroxidase-Konjugat und einem Streptavidin-Peroxidase-Konjugat.

16. Verfahren nach Anspruch 14, worin das Paar mit einem zweiten Antigen markiert ist, das mit dem Antikörper reagiert, und die sich an die Markierung bindende Substanz ein Konjugat aus einem Peroxidaseenzym und einem Antikörper für das zweite Antigen ist.

17. Verfahren nach einem der Ansprüche 1, 6, 7, 13 oder 14, worin das Licht in einer Reaktion erzeugt wird, in der das Peroxidaseenzym und das zyklische Hydroxyaryl-Diacylhydrazid in einer Zusammensetzung verwendet werden, die Folgendes umfasst:
das zyklische Hydroxyaryl-Diacylhydrazid; eine Phenolverbindung, die die Erzeugung von Licht aus dem Diacylhydrazid verstärkt und die Erzeugung von Licht aus dem Diacylhydrazid in Abwesenheit der Peroxidase reduziert; eine Peroxidverbindung, die mit dem Diacylhydrazid in Gegenwart der Peroxidase reagiert; einen Chelatbildner, der verhindert, dass die Peroxidverbindung das Diacylhydrazid vor der Zugabe der Peroxidase zu der Zusammensetzung aktiviert; und eine wasserlösliche oberflächenaktive Verbindung, die zur Verstärkung der Lichterzeugung dient.

18. Verfahren nach Anspruch 17, worin die wasserlösliche oberflächenaktive Verbindung eine nicht-ionische oberflächenaktive Substanz ist.

19. Verfahren nach Anspruch 17, worin die wasserlösliche oberflächenaktive Substanz ein kationisches Polymer ist.

20. Verfahren nach Anspruch 19, worin das kationische Polymer ein aus Poly(vinylbenzyl)trialkylphosphoniumsalzen ausgewähltes polymeres Phosphoniumsalz ist.

21. Verfahren nach Anspruch 19, worin das kationische Polymer ein aus Poly(vinylbenzyl)trialkylammoniumsalzen ausgewähltes polymeres Ammoniumsalz ist.

22. Verfahren nach einem der Ansprüche 1, 6, 7, 13, 14 oder 17, worin das zyklische Hydroxyaryl-Diacylhydrazid 5-Hydroxyphthalazin-1,4-dion ist.

23. Verfahren nach Anspruch 1, worin die Oberfläche ausgewählt ist aus der Gruppe bestehend aus Nylon-, Nitrozellulose- und Polyvinylidendifluorid-Membranen.

24. Verfahren nach einem der Ansprüche 1, 6, 7, 13, 14 oder 17, worin das erzeugte Licht auf einem photographischen Film nachgewiesen wird.

25. Kit zum Nachweis einer Nukleinsäure oder eines Fragments einer Nukleinsäure, das Folgendes umfasst:
(a) eine zu der Nukleinsäure oder einem Teil davon komplementäre, mit einem Peroxidaseenzym markierte Oligonukleotidsonde;
(b) eine zyklische Hydroxyaryl-Diacylhydrazid-Verbindung der Formel: 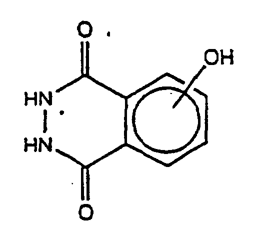 eine Phenolverstärkerverbindung in einer Menge, die die Lichterzeugung aus dem Diacylhydrazid in Gegenwart des Peroxidaseenzyms verstärkt und die Hintergrund-Chemolumineszenz reduziert;
(c) eine Peroxidaseverbindung in einer Menge, die mit dem Diacylhydrazid in Gegenwart der Peroxidase reagiert;
(d) einen Chelatbildner in einer Menge, die verhindert, dass die Peroxidverbindung das Diacylhydrazid vor der Reaktion mit der Peroxidase aktiviert; und eine wasserlösliche, die Chemolumineszenz verstärkende oberflächenaktive Substanz; sowie
(e) eine Membran, auf der die Hybridisierung erfolgt.

26. Kit zum Nachweis einer Nukleinsäure oder eines Fragments einer Nukleinsäure, das Folgendes umfasst:
(a) eine zu der Nukleinsäure oder einem Teil davon komplementäre, mit Biotin markierte Oligonukleotidsonde;
(b) ein Streptavidin-Peroxidase- oder Avidin-Peroxidase-Konjugat;
(c) eine zyklische Hydroxyaryl-Diacylhydrazid-Verbindung der Formel: 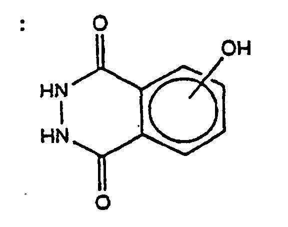 als Beimischung in einer wässrigen Lösung; eine Phenolverstärkerverbindung in einer Menge, die die Lichterzeugung aus dem Diacylhydrazid in Gegenwart der Peroxidase verstärkt und die Hintergrund-Chemolumineszenz reduziert; eine Peroxidverbindung in einer Menge, die mit dem Diacylhydrazid in Gegenwart der Peroxidase reagiert; einen Chelatbildner in einer Menge, die verhindert, dass die Peroxidverbindung das Diacylhydrazid vor der Reaktion mit der Peroxidase aktiviert; und eine wasserlösliche, die Chemolumineszenz verstärkende oberflächenaktive Substanz; sowie
(d) eine Membran, auf der die Hybridisierung erfolgt.

27. Kit zum Nachweis einer Nukleinsäure oder eines Fragments einer Nukleinsäure, das Folgendes umfasst:
(a) eine zu der Nukleinsäure oder einem Teil davon komplementäre, mit einem Antigen markierte Oligonukleotidsonde; ein Antikörper-Peroxidase-Konjugat, in dem der Antikörper auf das Antigen ausgerichtet ist;
(b) eine zyklische Hydroxyaryl-Diacylhydrazid-Verbindung der Formel: 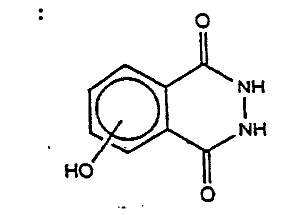 als Beimischung in einer wässrigen Lösung; eine Phenolverstärkerverbindung in einer Menge, die die Lichterzeugung aus dem Diacylhydrazid in Gegenwart der Peroxidase verstärkt und die Hintergrund-Chemolumineszenz reduziert; eine Peroxidverbindung in einer Menge, die mit dem Diacylhydrazid in Gegenwart der Peroxidase reagiert; einen Chelatbildner in einer Menge, die verhindert, dass die Peroxidverbindung das Diacylhydrazid vor der Reaktion mit der Peroxidase aktiviert; und eine wasserlösliche, die Chemolumineszenz verstärkende oberflächenaktive Substanz; sowie
(c) eine Membran, auf der die Hybridisierung erfolgt.

28. Kit zum Nachweis eines Proteins, das Folgendes umfasst:
(a) ein Antikörper-Peroxidase-Konjugat, in dem der Antikörper auf das Protein oder auf einen auf das Protein ausgerichteten primären Antikörper ausgerichtet ist;
(b) eine zyklische Hydroxyaryl-Diacylhydrazid-Verbindung der Formel: 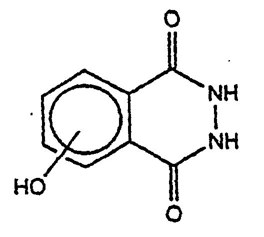 als Beimischung in einer wässrigen Lösung; eine Phenolverstärkerverbindung in einer Menge, die die Lichterzeugung aus dem Diacylhydrazid in Gegenwart der Peroxidase verstärkt und die Hintergrund-Chemolumineszenz reduziert; eine Peroxidverbindung in einer Menge, die mit dem Diacylhydrazid in Gegenwart der Peroxidase reagiert; einen Chelatbildner in einer Menge, die verhindert, dass die Peroxidverbindung das Diacylhydrazid vor der Reaktion mit der Peroxidase aktiviert; und eine wasserlösliche, die Chemolumineszenz verstärkende oberflächenaktive Substanz; sowie
(c) eine Membran, auf der die Protein-Antikörper-Verbindung erfolgt.

29. Kit zum Nachweis eines Proteins, das Folgendes umfasst:
(a) einen auf das Protein ausgerichteten primären Antikörper; ein auf den primären Antikörper ausgerichtetes Peroxidase-Sekundärantikörper-Konjugat;
(b) eine zyklische Hydroxyaryl-Diacylhydrazid-Verbindung der Formel: 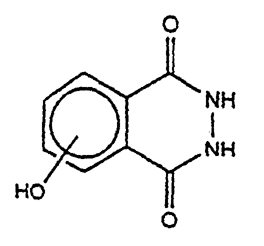 als Beimischung in einer wässrigen Lösung; eine Phenolverstärkerverbindung in einer Menge, die die Lichterzeugung aus dem Diacylhydrazid in Gegenwart der Peroxidase verstärkt und die Hintergrund-Chemolumineszenz reduziert; eine Peroxidverbindung in einer Menge, die mit dem Diacylhydrazid in Gegenwart der Peroxidase reagiert; einen Chelatbildner in einer Menge, die verhindert, dass die Peroxidverbindung das Diacylhydrazid vor der Reaktion mit der Peroxidase aktiviert; eine wasserlösliche, die Chemolumineszenz verstärkende oberflächenaktive Substanz sowie eine Membran, auf der die Protein-Antikörper-Verbindung erfolgt.

30. Kit nach einem der Ansprüche 25 bis 29, worin die zyklische Hydroxyaryl-Diacylhydrazid-Verbindung 5-Hydroxy-2,3-dihydrophthalazin-1,4-dion ist.

31. Kit nach einem der Ansprüche 25 bis 29, worin die wasserlösliche, die Chemolumineszenz verstärkende oberflächenaktive Substanz eine nicht-ionische oberflächenaktive Substanz ist.

32. Kit nach einem der Ansprüche 25 bis 29, worin die wasserlösliche, die Chemolumineszenz verstärkende oberflächenaktive Substanz ein kationisches Polymer ist.

33. Kit nach Anspruch 32, worin das kationische Polymer ein polymeres Phosphoniumsalz ist.

34. Kit nach Anspruch 32, worin das kationische Polymer ein Polyvinylbenzyltrialkylphosphoniumsalz ist.

35. Kit nach Anspruch 32, worin das kationische Polymer ein polymeres Ammoniumsalz ist.

36. Kit nach Anspruch 32, worin das kationische Polymer ein Polyvinylbenzyltrialkylammoniumsalz ist.

## Revendications

1. Procédé de détection d'un élément d'une paire liante spécifique marquée avec une enzyme peroxydase dans une procédure de dosage par une réaction chimioluminescente, comprenant :
(a) la réaction avec la paire liante sur une surface avec un diacylhydrazide cyclique d'hydroxyaryle de formule : 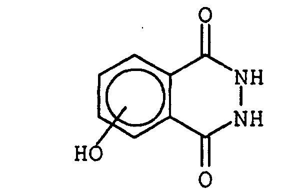
et un peroxyde, de façon à ce que de la lumière soit produite pour détecter l'analyte.

2. Procédé selon la revendication 1, dans lequel la paire liante spécifique est constituée d'un acide nucléique et d'une sonde oligonucléotidique qui est complémentaire de et se fixe à l'acide nucléique cible ou à une partie de celui-ci, dans lequel la sonde est associée à l'enzyme peroxydase par le biais d'une liaison covalente, d'attractions physiques ou par liaison par l'intermédiaire d'une paire liante spécifique marquée par une enzyme.

3. Procédé selon, la revendication 2, dans lequel l'acide nucléique est l'ADN.

4. Procédé selon la revendication 2, dans lequel l'acide nucléique est l'ARN.

5. Procédé selon la revendication 2, dans lequel l'enzyme peroxydase est la peroxydase de raifort.

6. Procédé selon la revendication 2, constitué en outre des étapes consistant à (a) hybrider l'acide nucléique et la sonde oligonucléotidique directement marquée avec la peroxyde de raifort pour former la paire liante, et (b) faire réagir la paire liante avec le diacylhydrazide cyclique d'hydroxyaryle et un peroxyde, de façon à ce que de la lumière soit produite.

7. Procédé selon la revendication 2, constitué en outre des étapes consistant à (a) hybrider l'acide nucléique et la sonde oligonucléotidique marquée pour former la paire liante, (b) faire réagir la paire liante avec une substance qui se fixe fortement à la paire et qui est fixée à l'enzyme peroxydase pour former un conjugué enzymatique fixé, et (c) faire réagir le conjugué enzymatique fixé avec un diacylhydrazide cyclique d'hydroxyaryle et un peroxyde, de façon à ce que de la lumière soit produite.

8. Procédé selon la revendication 7, dans lequel la paire liante contient de la biotine et la substance est choisie dans le groupe constitué par un conjugué avidine-peroxydase de raifort et un conjugué streptavidine-peroxydase.

9. Procédé selon la revendication 7, dans lequel la paire contient un site antigénique et la substance est un conjugué anticorps-peroxydase qui se fixe au site.

10. Procédé selon la revendication 1, dans lequel la paire liante spécifique est constituée d'un antigène et d'un anticorps qui se fixe à l'antigène.

11. Procédé selon la revendication 10, dans lequel l'antigène ou l'anticorps est associé à une enzyme peroxydase par le biais d'une liaison covalente, d'attractions physiques, ou par liaison par l'intermédiaire d'une paire liante spécifique marquée par une enzyme.

12. Procédé selon la revendication 11, dans lequel l'enzyme est la peroxydase de raifort.

13. Procédé selon la revendication 12, constitué en outre des étapes consistant à (a) former un complexe antigène-anticorps et (b) faire réagir le complexe antigène-anticorps marqué avec une enzyme peroxydase avec le diacylhydrazide cyclique d'hydroxyaryle et un peroxyde, de façon à ce que de la lumière soit produite.

14. Procédé selon la revendication 11, constitué en outre des étapes consistant à (a) former un complexe antigène-anticorps ; et (b) faire réagir le complexe antigène-anticorps marqué avec une substance qui se fixe fortement à la paire et est fixée à une enzyme peroxydase, et (c) faire réagir le conjugué enzymatique fixé avec le diacylhydrazide cyclique d'hydroxyaryle et un peroxyde, de façon à ce que de la lumière soit produite.

15. Procédé selon la revendication 14, dans lequel la paire est marquée avec de la biotine et la substance est choisie dans le groupe constitué par un conjugué avidine-peroxydase et un conjugué streptavidine-peroxydase.

16. Procédé selon la revendication 14, dans lequel la paire est marquée avec un second antigène qui réagit avec l'anticorps et la substance fixant le traceur est un conjugué entre une enzyme peroxydase et un anticorps dirigé contre le second antigène.

17. Procédé selon l'une quelconque des revendications 1, 6, 7, 13 ou 14, dans lequel la lumière est produite dans une réaction utilisant l'enzyme peroxydase et le diacylhydrazide cyclique d'hydroxyaryle dans une composition qui comprend :
le diacylhydrazide cyclique d'hydroxyaryle ; un composé phénolique qui active la production de lumière à partir du diacylhydrazide et diminue la production de lumière à partir du diacylhydrazide en l'absence de peroxydase ; un composé de type peroxyde qui réagit avec le diacylhydrazide en présence de la peroxydase ; un agent chélateur qui empêche le composé de type peroxyde d'activer le diacylhydrazidé avant l'ajout de la peroxydase dans la composition ; et un composé tensioactif soluble dans l'eau ayant pour fonction d'activer la production de lumière.

18. Procédé selon la revendication 17, dans lequel le composé tensioactif soluble dans l'eau est un tensioactif non ionique.

19. Procédé selon la revendication 17, dans lequel le composé tensioactif soluble dans l'eau est un polymère cationique.

20. Procédé selon la revendication 19, dans lequel le polymère cationique est un sel de phosphonium polymère choisi parmi les sels de poly(vinylbenzyl)-trialkylphosphonium.

21. Procédé selon la revendication 19, dans lequel le polymère cationique est un sel d'ammonium polymère choisi parmi les sels de poly(vinylbenzyl)-trialkylammonium.

22. Procédé selon l'une quelconque des revendications 1, 6, 7, 13, 14 ou 17, dans lequel le diacylhydrazide cyclique d'hydroxyaryle est le 5-hydroxyphtalazine-1,4-dione.

23. Procédé selon la revendication 1, dans lequel la surface est choisie dans le groupe constitué par les membranes en nylon, nitrocellulose et difluorure de polyvinylidène.

24. Procédé selon l'une quelconque des revendications 1, 6, 7, 13, 14 ou 17, dans lequel la lumière produite est détectée sur un film photographique.

25. Trousse de détection d'un acide nucléique ou d'un fragment d'acide nucléique comprenant :
(a) une sonde oligonucléotidique complémentaire de l'acide nucléique ou d'une partie de celui-ci marquée avec une enzyme peroxydase ;
(b) un composé de type diacylhydrazide cyclique d'hydroxyaryle de formule : 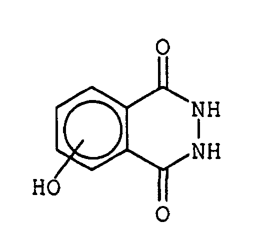 un composé de type activateur phénolique dans une quantité qui active la production de lumière à partir du diacylhydrazide en présence de l'enzyme peroxyde et diminue la chimiluminescence de fond ;
(c) un composé de type peroxyde dans une quantité qui réagit avec le diacylhydrazide en présence de la peroxydase ;
(d) un agent chélateur dans une quantité qui empêche le composé de type peroxyde d'activer le diacylhydrazide avant la réaction avec la peroxydase ; et un tensioactif activant la chimiluminescence soluble dans l'eau ; et
(e) une membrane sur laquelle l'hybridation est effectuée.

26. Trousse de détection d'un acide nucléique ou d'un fragment d'acide nucléique comprenant :
(a) une sonde oligonucléotidique complémentaire de l'acide nucléique ou d'une partie de celui-ci marquée avec de la biotine ;
(b) un conjugué streptavidine-peroxydase ou avidine-peroxydase ;
(c) en mélange dans une solution aqueuse, un composé de type diacylhydrazide cyclique d'hydroxyaryle de formule : 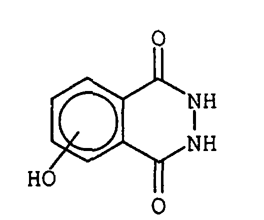 un composé de type activateur phénolique dans une quantité qui active la production de lumière à partir du diacylhydrazide en présence de la peroxyde et diminue la chimiluminescence de fond ; un composé de type peroxyde dans une quantité qui réagit avec le diacylhydrazide en présence de la peroxydase ; un agent chélateur dans une quantité qui empêche le composé de type peroxyde d'activer le diacylhydrazide avant la réaction avec la peroxydase ; et un tensioactif activant la chimiluminescence soluble dans l'eau ; et
(d) une membrane sur laquelle l'hybridation est effectuée.

27. Trousse de détection d'un acide nucléique ou d'un fragment d'acide nucléique comprenant :
(a) une sonde oligonucléotidique complémentaire de l'acide nucléique ou d'une partie de celui-ci marquée avec un antigène ; un conjugué anticorps-peroxydase dans lequel l'anticorps est dirigé contre l'antigène ;
(b) en mélange dans une solution aqueuse, un composé de type diacylhydrazide cyclique d'hydroxyaryle de formule : 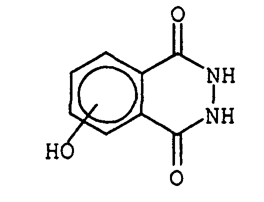 un composé de type activateur phénolique dans une quantité qui active la production de lumière à partir du diacylhydrazide en présence de la peroxydase et diminue la chimiluminescence de fond ; un composé de type peroxyde dans une quantité qui réagit avec le diacylhydrazide en présence de la peroxydase ; un agent chélateur dans une quantité qui empêche le composé de type peroxyde d'activer le diacylhydrazide avant la réaction avec la peroxydase ; et un tensioactif activant la chimiluminescence soluble dans l'eau ; et
(c) une membrane sur laquelle l'hybridation est effectuée.

28. Trousse de détection d'une protéine comprenant :
(a) un conjugué anticorps-peroxydase dans lequel l'anticorps est dirigé contre la protéine ou contre un anticorps primaire dirigé contre la protéine ;
(b) en mélange dans une solution aqueuse, un composé de type diacylhydrazide cyclique d'hydroxyaryle de formule : 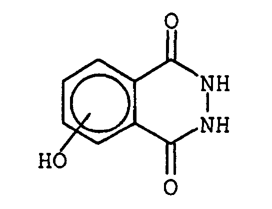 un composé de type activateur phénolique dans une quantité qui active la production de lumière à partir du diacylhydrazide en présence de la peroxydase et diminue la chimiluminescence de fond ; un composé de type peroxyde dans une quantité qui réagit avec le diacylhydrazide en présence de la peroxydase ; un agent chélateur dans une quantité qui empêche le composé de type peroxyde d'activer le diacylhydrazide avant la réaction avec la peroxydase ; un tensioactif activant la chimiluminescence soluble dans l'eau ; et
(c) une membrane sur laquelle la liaison protéine-anticorps est effectuée.

29. Trousse de détection d'une protéine comprenant :
(a) un anticorps primaire dirigé contre la protéine ; un conjugué peroxydase-anticorps secondaire dirigé contre l'anticorps primaire ;
(b) en mélange dans une solution aqueuse, un composé de type diacylhydrazide cyclique d'hydroxyaryle de formule : 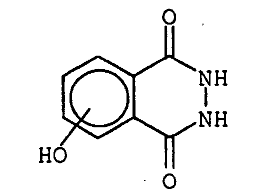 un composé de type activateur phénolique dans une quantité qui active la production de lumière à partir du diacylhydrazide en présence de la peroxydase et diminue la chimiluminescence de fond ; un composé de type peroxyde dans une quantité qui réagit avec le diacylhydrazide en présence de la peroxydase ; un agent chélateur dans une quantité qui empêche le composé de type peroxyde d'activer le diacylhydrazide avant la réaction avec la peroxydase ; un tensioactif activant la chimiluminescence soluble dans l'eau et une membrane sur laquelle la liaison protéine-anticorps est effectuée.

30. Trousse selon l'une quelconque des revendications 25 à 29, dans laquelle le composé de type diacylhydrazide cyclique d'hydroxyaryle est le 5-hydroxy-2,3-dihydrophtalazine-1,4-dione.

31. Trousse selon l'une quelconque des revendications 25 à 29, dans laquelle le tensioactif activant la chimiluminescence soluble dans l'eau est un tensioactif non ionique.

32. Trousse selon l'une quelconque des revendications 25 à 29, dans laquelle le tensioactif activant la chimiluminescence soluble dans l'eau est un polymère cationique.

33. Trousse selon la revendication 32, dans laquelle le polymère cationique est un sel de phosphonium polymère.

34. Trousse selon la revendication 32, dans laquelle le polymère cationique est un sel de polyvinylbenzyl-trialkylphosphonium.

35. Trousse selon la revendication 32, dans laquelle le polymère cationique est un sel d'ammonium polymère.

36. Trousse selon la revendication 32, dans laquelle le polymère cationique est un sel de polyvinylbenzyl-trialkylammonium.
